# EUROPEAN PATENT SPECIFICATION

(11) **EP 1 446 404 B1**
(45) Date of publication and mention of the grant of the patent: **14.06.2006**
(21) Application number: 02779713.3
(22) Date of filing: 21.11.2002
(51) Int. Cl.: C07D 417/12, A61K 31/4439, A61P 3/10

(54) **ROSIGLITAZONE EDISYLATES AND THEIR USE AS ANTIDIABETICS**
ROSIGLITAZON-EDISYLATE UND IHRE VERWENDUNG ALS ANTIDIABETIKA
EDISYLATES DE ROSIGLITAZONE ET LEUR UTILISATION EN TANT QU'ANTIDIABETIQUES

(30) Priority: 21.11.2001 GB 0127931; 21.11.2001 GB 0127932; 21.11.2001 GB 0127933
(43) Date of publication of application: 18.08.2004
(73) Proprietor: SMITHKLINE BEECHAM PLC, Brentford, Middlesex TW8 9GS (GB)
(72) Inventor: HO, Tim, Chien, Ting, GlaxoSmithKline, Tonbridge, Kent TN11 9AN (GB); MILLAN, Michael, John, GlaxoSmithKline, Tonbridge, Kent TN11 9AN (GB)
(74) Representative: Rutter, Keith
(86) International application number: PCT/GB2002/005239
(87) International publication number: WO 2003/045947

(56) References cited:
- WO-A-94/05659
- US-A- 4 108 994
- BERGE S M ET AL: "Pharmaceutical salts" JOURNAL OF PHARMACEUTICAL SCIENCES, vol. 66, no. 1, 1977, pages 1-19, XP000562636

## Description

This invention relates to a novel compound, in particular to a novel pharmaceutical being a novel salt of a certain thiazolidinedione, to a process for the preparation of the said compound and to the use of the compound in medicine.

EP-A-0 306 228 relates to certain thiazolidinedione derivatives disclosed as having hypoglycaemic and hypolipidaemic activity. The compound of Example 30 of EP-A-0 306 228 is 5-[4-[2-(N-methyl-N-(2-pyridyl)amino)ethoxy]benzyl]thiazolidine-2,4-dione (hereinafter referred to as "Compound (I)").

WO 94/05659 discloses certain salts of the compounds of EP-A-0 306 228. The preferred salt of WO 94/05659 is the maleic acid salt.

We have now prepared 1,2-ethanedisulfonate salts of Compound (I) (for convenience sometimes referred hereinafter by the abbreviation "Ethanedisulfonate") and characterised an Ethandisulphonate that is particularly stable and hence is suitable for bulk preparation and handling. The Ethanedisulfonate also has a high melting point, shows particularly good aqueous solubility and possesses good bulk flow properties. The Ethanedisulfonate is therefore surprisingly amenable to large scale pharmaceutical processing and especially to large scale milling.

Ethanedisulfonate salts of other pharmaceutically active agents are known. See, for example, Berge et al., J. Pharm. Sci. 66(1) 1-19 (1977) and US-A-4108994.

The novel Ethanedisulfonate can be prepared by an efficient and economic process particularly suited to large-scale preparation.

The novel Ethanedisulfonate also has useful pharmaceutical properties and in particular it is indicated to be useful for the treatment and/or prophylaxis of diabetes mellitus, conditions associated with diabetes mellitus and certain complications thereof.

Accordingly, the present invention provides 5-[4-[2-(N-methyl-N-(2-pyridyl)amino)ethoxy]benzyl]thiazolidine-2,4-dione ethanedisulfonate salt (the Ethanedisulphonate), or a solvate thereof.

Ethanedisulfonic acid is a diacid, thus the Ethanedisulfonate of the invention can form either a 1:1, Compound (I): ethanedisulfonate molar ratio salt or a 2:1, Compound (I): ethanedisulfonate molar ratio salt. In its preferred aspect, the present invention provides an Ethanedisulfonate in which the molar ratio of Compound (I) to ethanedisulfonic acid is 2:1 (the 2:1 salt). However an Ethanedisulfonate in which the molar ratio of Compound (I) to ethanedisulfonic acid is 1:1 forms another aspect of the invention (the 1:1 salt).

In the 1:1 salt, the ethanedisulfonate anion may be associated with a proton (hydrogen atom) in addition to Compound (I) or may be associated with another cation, for example an alkali metal or ammonium cation. In the former case the 1:1 salt may be described as a hydrogen ethanedisulfonate, while in the latter case the salt may be described as a mixed salt.

The Ethanedisulfonate of the invention extends to hydrogen ethanedisulphonates.

The Ethanedisulfonate of the invention also extends to mixed salts.

It has also been found that the Ethanedisulfonate 2:1 salt exists in more than one polymorphic form. The present invention extends to all polymorphic forms of the Ethanedisulphonate whether in a pure polymorphic form or when admixed with any other material, such as another polymorphic form. Herein, certain novel polymorphic forms of the Ethanedisulfonate 2:1 salt are referred to as Form I and Form II.

Suitably, the invention provides the Ethanedisulfonate, or a solvate thereof characterised by spectral data, including at least one of, infra red, Raman, X-ray, (carbon or hydrogen) nuclear magnetic resonance spectral data and/or melting point data.

In a further aspect the invention provides Ethanedisulfonate Form I, or a solvate thereof.

In a further aspect the invention provides Ethanedisulfonate Form II, or a solvate thereof.

In a particular aspect, the present invention provides 5-[4-[2-(N-methyl-N-(2-pyridyl)amino)ethoxy]benzyl]thiazolidine-2,4-dione ethanedisulfonate salt Form I (Ethanedisulphonate Form I), characterised in that it provides:
(i) an infra red spectrum containing peaks at about 1303, 1214, 1026 and 763 cm⁻¹; and/or
(ii) a Raman spectrum containing peaks at about 1415, 1323, 1053, and 293 cm⁻¹; and/or
(iii) an X-ray powder diffraction (XRPD) pattern containing peaks at about 9.2, 11.0, 13.3, 15.5, 17.2 and 18.0 °2θ.

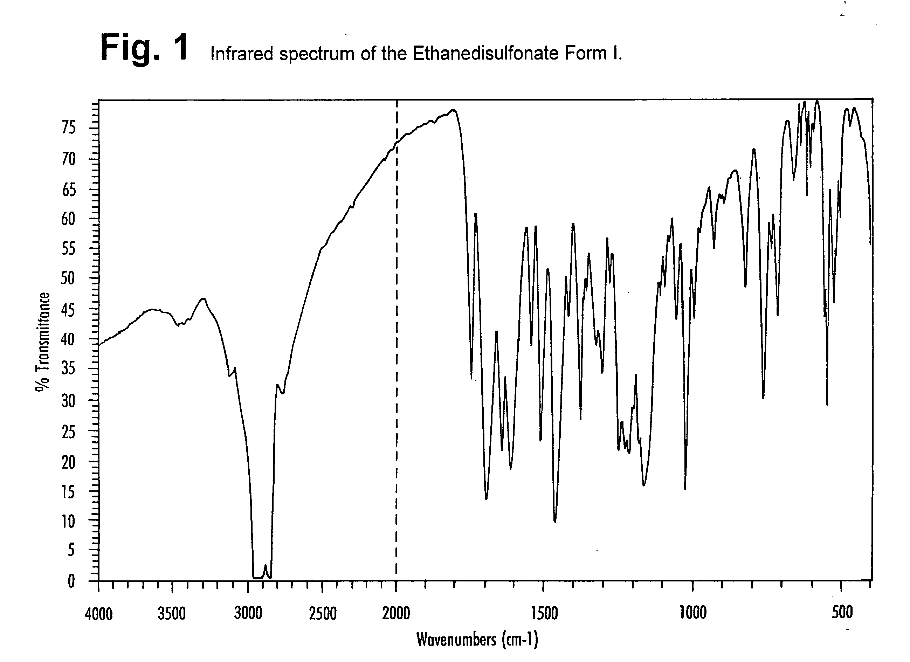

In one favoured aspect, the Ethanedisulfonate Form I provides an infrared spectrum substantially in accordance with Figure 1.

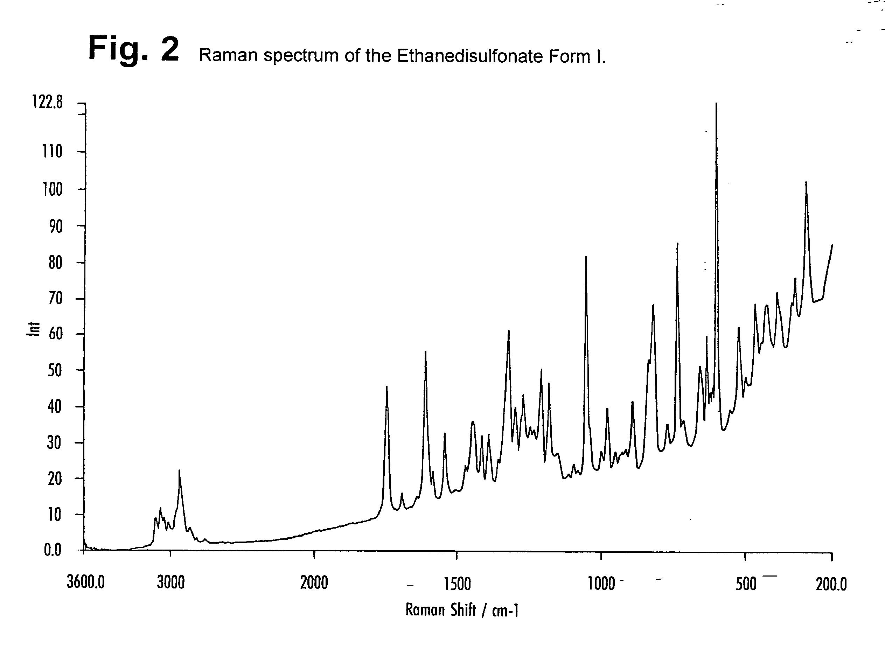

In one favoured aspect, the Ethanedisulfonate Form I provides a Raman spectrum substantially in accordance with Figure 2.

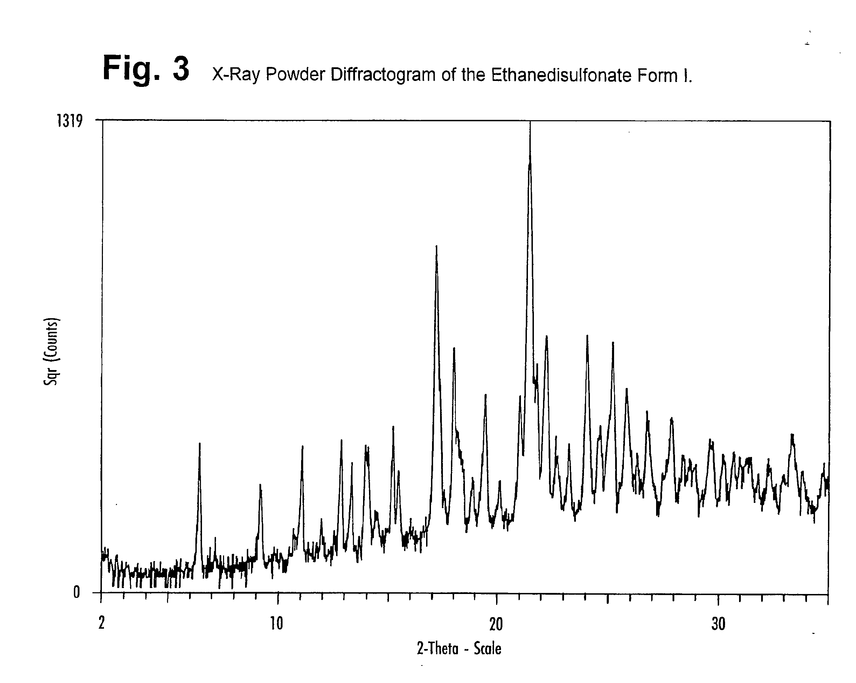

In one favoured aspect, the Ethanedisulfonate Form I provides an X-Ray powder diffraction pattern (XRPD) substantially in accordance with Table 1 or Figure 3.

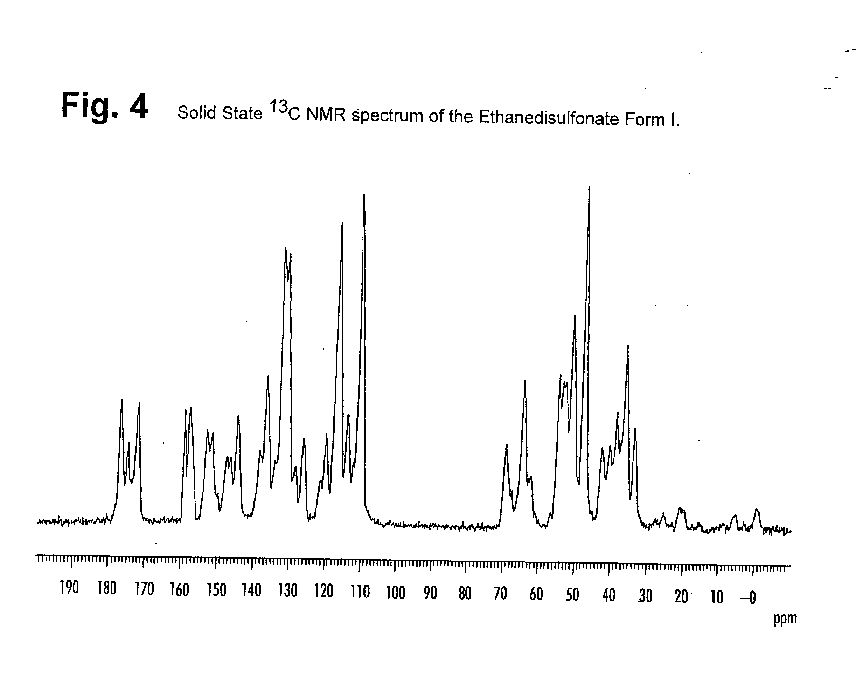

In one favoured aspect, the Ethanedisulfonate Form I provides a Solid State ¹³C NMR spectrum substantially in accordance with Figure 4.

In a particular aspect, the present invention provides 5-[4-[2-(N-methyl-N-(2-pyridyl)amino)ethoxy]benzyl]thiazolidine-2,4-dione ethanedisulfonate salt Form II (Ethanedisulfonate Form II), characterised in that it provides:
(i) an infra red spectrum containing peaks at about 1315, 1274, 1022, 904 and 513 cm⁻¹; and/or
(ii) a Raman spectrum containing peaks at about 1706, 1645, 1382, 1327 and 901 cm⁻¹ ; and/or
(iii) an X-ray powder diffraction (XRPD) pattern containing peaks at about 12.6, 14.8, 19.9 and 23.7 °2θ.

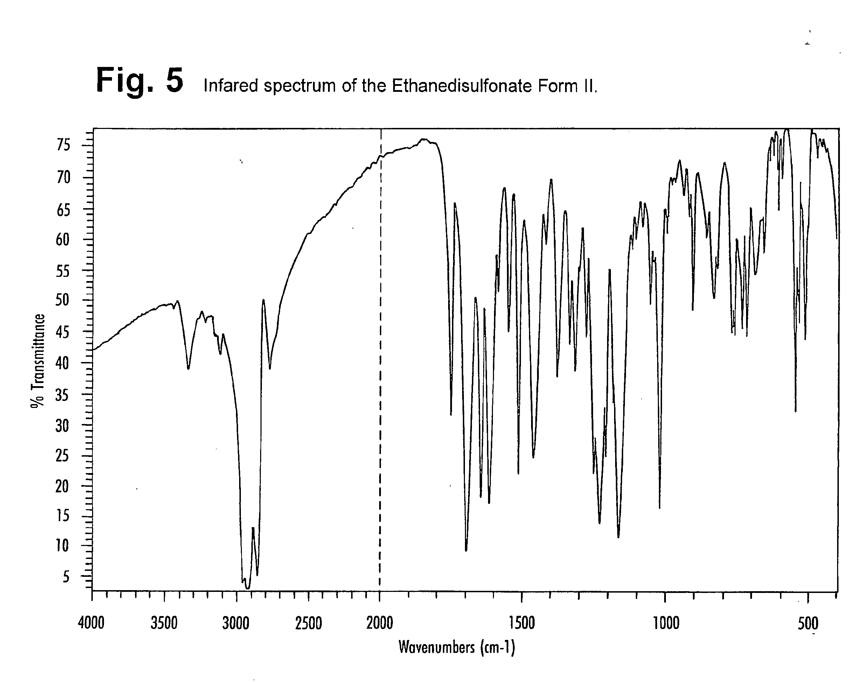

In one favoured aspect, the Ethanedisulfonate Form II provides an infrared spectrum substantially in accordance with Figure 5.

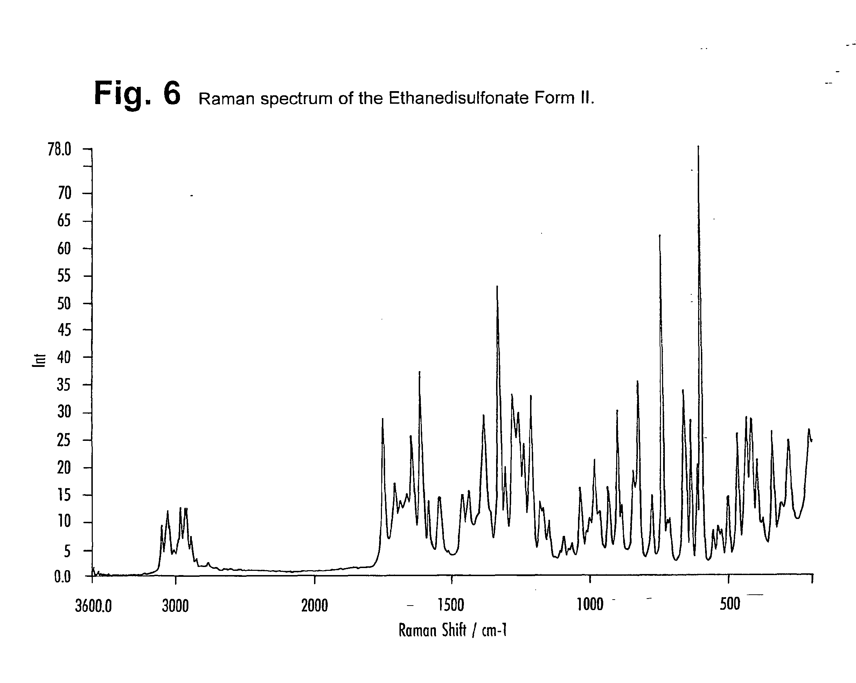

In one favoured aspect, the Ethanedisulfonate Form II provides a Raman spectrum substantially in accordance with Figure 6.

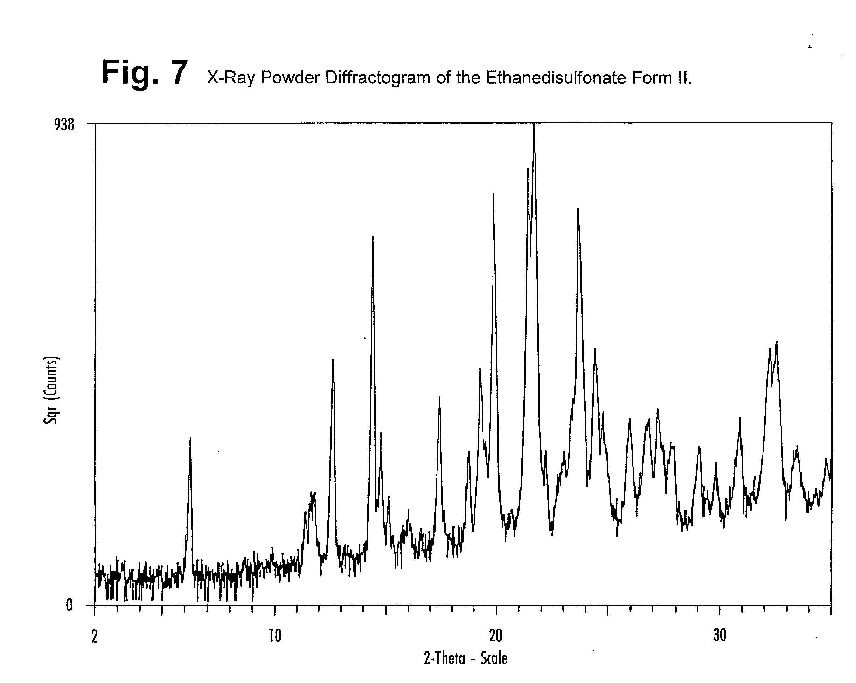

In one favoured aspect, the Ethanedisulfonate Form II provides an X-Ray powder diffraction pattern (XRPD) substantially in accordance with Table 2 or Figure 7.

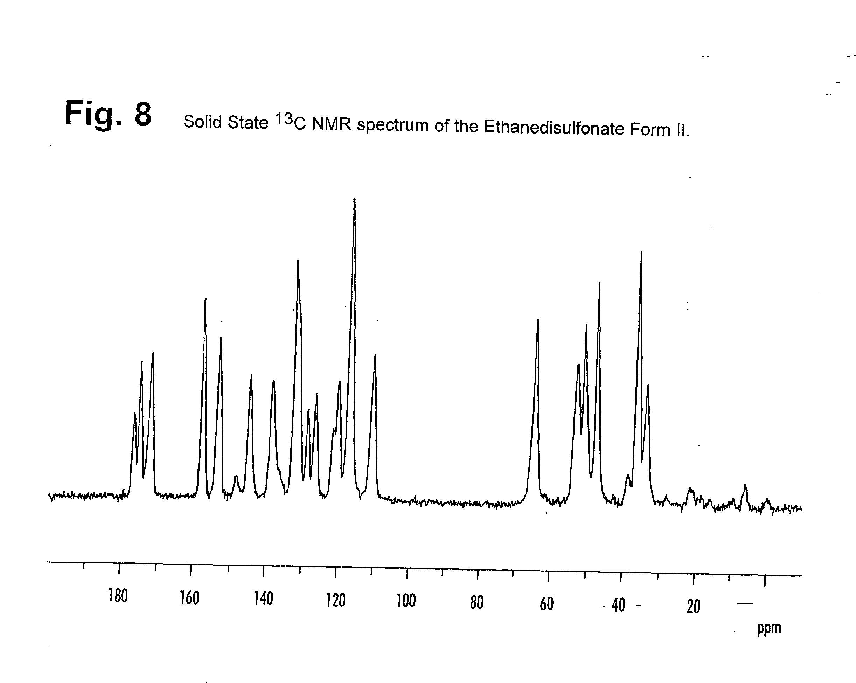

In one favoured aspect, the Ethanedisulfonate Form II provides a Solid State ¹³C NMR spectrum substantially in accordance with Figure 8.

In a further favoured aspect the Ethanedisulfonate Form II, provides a melting range in the range of 192 to 205 °C, for example 197.8 -199.0°C.

In a most preferred aspect, the invention provides 5-[4-[2-(N-methyl-N-(2-pyridyl)amino)ethoxy]benzyl]thiazolidine-2,4-dione, ethanedisulfonate salt Form II, characterised in that it provides:
(i) an infrared spectrum substantially in accordance with Figure 5; and
(ii) a Raman spectrum substantially in accordance with Figure 6; and
(iii) an X-Ray powder diffraction pattern (XRPD) substantially in accordance with Table 2 or Figure 7; and
(iv) a Solid State ¹³C NMR spectrum substantially in accordance with Figure 8.

The present invention encompasses the Ethanedisulfonate or a solvate thereof isolated in a purified form or when a admixed with other materials.

Thus in one aspect there is provided the Ethanedisulfonate or a solvate thereof in an isolated form, that is isolated substantially from any impurity. Thus in a particular aspect there is provided the Ethanedisulfonate or a solvate thereof in substantially pure form.

In yet a further aspect there is provided the Ethanedisulfonate or a solvate thereof in crystalline form.

Also, the invention provides the Ethanedisulfonate or solvate thereof in a solid pharmaceutically acceptable form, such as a solid dosage form, especially when adapted for oral administration.

Moreover, the invention also provides the Ethanedisulfonate or solvate thereof in a pharmaceutically acceptable form, especially in bulk form, such form being particularly capable of being milled. The invention therefor also provides the Ethanedisulfonate or solvate thereof in a milled form.

Furthermore, the invention provides the Ethanedisulfonate or solvate thereof in a pharmaceutically acceptable form, especially in bulk form, such form having good flow properties, especially good bulk flow properties.

Solvates include pharmaceutically acceptable solvates, such as hydrates. A suitable solvate is a hydrate.

The invention also provides a process for preparing the Ethanedisulfonate or a solvate thereof, characterised in that 5-[4-[2-(N-methyl-N-(2-pyridyl)amino)ethoxy]benzyl]thiazolidine-2,4-dione (Compound (I)) or a salt thereof, preferably dispersed or dissolved in a suitable solvent, is reacted with a suitable source of ethanedisulfonate ion; and the Ethanedisulfonate or a solvate thereof is recovered.

Ethanedisulfonates may be prepared by contacting appropriate amounts, such as stoichiometric amounts (1:1 or 1:2), of the acid and Compound (I); alternatively a relative excess of the acid may be used. Mixed salts may be prepared by forming a precursor 1:1 salt *in situ* or using it pre-formed; and contacting the precursor salt with a solution containing the metal or ammonium ion, or treating a metal or ammonium hydrogen ethanedisulfonate with Compound (I).

A suitable reaction solvent is an alkanol, for example propan-2-ol, or a hydrocarbon, such as toluene, a ketone, such as acetone, an ester, such as ethyl acetate, an ether such as tetrahydrofuran, a nitrile such as acetonitrile, or a halogenated hydrocarbon such as dichloromethane or water, or an organic acid such as acetic acid; or a mixture thereof.

Conveniently, the source of ethanedisulfonate ion is ethanedisulfonic acid. The ethanedisulfonic acid is preferably added as a solid or in solution, for example in water, ether, ketone, nitrile or a lower alcohol such as methanol, ethanol, or propan-2-ol, or a mixture of solvents. For example, a solution of ethanedisulfonic acid in IMS (industrial methylated spirits), ethanol or propan-2-ol may be added to a solution of Compound (I) also in the same solvent.

An alternative source of ethanedisulfonate ion is provided by a base salt of ethanedisulfonic acid for example ammonium ethanedisulfonate, or the ethanedisulfonic acid salt of an amine, for example ethylamine or diethylamine.

The concentration of Compound (1) is preferably in the range 3 to 50% weight/volume, more preferably in the range 5 to 20%. The concentration of ethanedisulfonic acid solutions are preferably in the range of 5 to 100% weight/volume.

The reaction is usually carried out at ambient temperature or at an elevated temperature, for example at the reflux temperature of the solvent, although any convenient temperature that provides the required product may be employed.

Solvates, such as hydrates, of the Ethanedisulfonate are prepared according to conventional procedures, for example by crystallising or recrystallising from a solvent which provides or contains the solvate moiety, or by exposing the Ethanedisulfonate to the solvate moiety as a vapour.

Recovery of the required compound generally comprises crystallisation from an appropriate solvent, conveniently the reaction solvent, usually assisted by cooling. For example, the Ethanedisulfonate may be crystallised from an alcohol such as ethanol or propan-2-ol, an ester such as ethyl acetate, an ether such as tetrahydrofuran, a nitrile such as acetonitrile, or water; or a mixture thereof. An improved yield of the salt can be obtained by evaporation of some or all of the solvent or by crystallisation at elevated temperature followed by controlled cooling. Careful control of precipitation temperature and seeding may be used to improve the reproducibility of the product form.

Crystallisation can also be initiated by seeding with crystals of the Ethanedisulfonate or a solvate thereof but this is not essential unless mentioned to the contrary herein.

In our hands during preparation of the 2:1 salt, either Ethanedisulfonate Form I or Ethanedisulfonate II or mixtures thereof, is produced by the above mentioned process. Thus, to ensure formation of a particular polymorph in substantially pure form the crystallisation step is preferably seeded with seeds of the required polymorph. Accordingly, Ethanedisulfonate Form I is suitably crystallised by seeding with seeds of Ethanedisulfonate Form I. Ethanedisulfonate Form II is suitably crystallised by seeding with seeds of Ethanedisulfonate Form II.

In one aspect the Ethanedisulfonate is Ethanedisulfonate Form I.

In one aspect the Ethanedisulfonate is Ethanedisulfonate Form I together with Ethanedisulfonate Form II .

Preferably, the Ethanedisulfonate is Ethanedisulfonate Form II. Thus in its preferred form, the Ethanedisulfonate is (2:1 salt) Ethanedisulfonate Form II.
Compound (I) is prepared according to known procedures, such as those disclosed in EP-A-0 306 228 and WO 94/05659.

1,2-Ethanedisulfonic acid is a commercially available compound.

When used herein "Ethanedisulphonate" refers to any and all polymorphic forms of 5-[4-[2-(N-methyl-N-(2-pyridyl)amino)ethoxy]benzyl]thiazolidine-2,4-dione ethanedisulfonate salt unless clarifed herein to the contrary.

When used herein the term "Tₒₙₛₑₜ" is generally determined by Differential Scanning Calorimetry and has a meaning generally understood in the art, as for example expressed in Pharmaceutical Thermal Analysis, Techniques and Applications", Ford and Timmins, 1989 as "The temperature corresponding to the intersection of the pre-transition baseline with the extrapolated leading edge of the trausition".

When used herein in respect of certain compounds the term "good flow properties" is suitably characterised by the said compound having a Hausner ratio of less than or equal to 1.5, especially of less than or equal to 1.25.

"Hausner ratio" is an art accepted term.

When used herein the term 'prophylaxis of conditions associated with diabetes mellitus' includes the treatment of conditions such as insulin resistance, impaired, glucose tolerance, hyperinsulinaemia and gestational diabetes.

Diabetes mellitus preferably means Type II diabetes mellitus.

Conditions associated with diabetes include hyperglycaemia and insulin resistance and obesity. Further conditions associated with diabetes include hypertension, cardiovascular disease, especially atherosclerosis, certain eating disorders, in particular the regulation of appetite and food intake in subjects suffering from disorders associated with under-eating, such as anorexia nervosa, and disorders associated with over-eating, such as obesity and anorexia bulimia. Additional conditions associated with diabetes include polycystic ovarian syndrome and steroid induced insulin resistance.

The complications of conditions associated with diabetes mellitus encompassed herein includes renal disease, especially renal disease associated with the development of Type II diabetes including diabetic nephropathy, glomerulonephritis, glomerular sclerosis, nephrotic syndrome, hypertensive nephrosclerosis and end stage renal disease.

As mentioned above the compound of the invention has useful therapeutic properties: The present invention accordingly provides the Ethanedisulfonate or a solvate thereof for use as an active therapeutic substance.

More particularly, the present invention provides the Ethanedisulfonate or a solvate thereof for use in the treatment and/or prophylaxis of diabetes mellitus, conditions associated with diabetes mellitus and certain complications thereof

The Ethanedisulfonate or a solvate thereof may be administered per se or, preferably, as a pharmaceutical composition also comprising a pharmaceutically acceptable carrier. Suitable methods for formulating the Ethanedisulfonate or a solvate thereof are generally those disclosed for Compound (I) in the above mentioned publications.

Accordingly, the present invention also provides a pharmaceutical composition comprising the Ethanedisulfonate or a solvate thereof and a pharmaceutically acceptable carrier therefor.

The Ethanedisulfonate or a solvate thereof is normally administered in unit dosage form.

The active compound may be administered by any suitable route but usually by the oral or parenteral routes. For such use, the compound will normally be employed in the form of a pharmaceutical composition in association with a pharmaceutical carrier, diluent and/or excipient, although the exact form of the composition will naturally depend on the mode of administration.

Compositions are prepared by admixture and are suitably adapted for oral, parenteral or topical administration, and as such may be in the form of tablets, capsules, oral liquid preparations, powders, granules, lozenges, pastilles, reconstitutable powders, injectable and infusable solutions or suspensions, suppositories and transdermal devices. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulfate.

Solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the active compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the active compound is suspended in the vehicle instead of being dissolved and sterilised by exposure to ethylene oxide before suspending in the sterile vehicle. Advantageously, a surfactant or wetting agent is included in the composition to facilitate uniform distribution of the active compound.

As is common practice, the compositions will usually be accompanied by written or printed directions for use in the medical treatment concerned.

As used herein the term 'pharmaceutically acceptable' embraces compounds, compositions and ingredients for both human and veterinary use: for example the term 'pharmaceutically acceptable salt' embraces a veterinarily acceptable salt.

The compositions are formulated according to conventional methods, such as those disclosed in standard reference texts, for example the British and US Pharmacopoeias, Remington's Pharmaceutical Sciences (Mack Publishing Co.), Martindale The Complete Drug Reference (London, The Pharmaceutical Press) and Harry's Cosmeticology (Leonard Hill Books).

Conveniently, the active ingredient may be administered as a pharmaceutical composition hereinbefore defined, and this forms a particular aspect of the present invention.

In a further aspect the present invention provides the use of the Ethanedisulfonate or a solvate thereof for the manufacture of a medicament for the treatment and/or prophylaxis of diabetes mellitus, conditions associated with diabetes mellitus and certain complications thereof.

In the treatment and/or prophylaxis of diabetes mellitus, conditions associated with diabetes mellitus and certain complications thereof, the Ethanedisulfonate or a solvate thereof may be taken in amounts so as to provide Compound (I) in suitable doses, such as those disclosed in EP-A-0 306 228, WO 94/05659 or WO 98/55122.

The unit dose compositions of the invention comprise the Ethanedisulfonate in an amount providing up to 12mg, including 1-12mg such as 2-12mg of Compound (I), especially 2-4mg, 4-8mg or 8-12mg of Compound (I), for example 1, 2, 3, 4, 8, 4 to 8 or 8 to 12mg of Compound (I). Thus in particular there is provided a pharmaceutical composition comprising the Ethanedisulfonate or a solvate thereof and a pharmaceutically acceptable carrier therefor, wherein the Ethanedisulfonate or a pharmaceutically acceptable solvate thereof is present in an amount providing 1, 2, 3, 4, 8, 4 to 8 or 8 to 12mg of Compound (I); such as 1mg of Compound (I); such as 2mg of Compound (I); such as 3mg of Compound (I); such as 4 of Compound (I); such as 8mg of Compound (I); such as 12mg of Compound (I);

The invention also provides a pharmaceutical composition comprising the Ethanedisulfonate or a pharmaceutically acceptable solvate thereof in combination with one or more other medicaments such as anti-diabetic agents and optionally a pharmaceutically acceptable carrier therefor.

In a further aspect the present invention provides the use of the Ethanedisulfonate or a pharmaceutically acceptable solvate thereof in combination with one or more other anti-diabetic agents, for the manufacture of a medicament for the treatment and/or prophylaxis of diabetes mellitus, conditions associated with diabetes mellitus and certain complications thereof.

In the above mentioned treatments the administration of the Ethanedisulfonate or a pharmaceutically acceptable solvate thereof and the other anti-diabetic agent or agents includes co-administration or sequential administration of the active agents.

Suitably in the above mentioned compositions, including unit doses, or treatments the Ethanedisulfonate or a pharmaceutically acceptable solvate thereof is present in an amount providing up to 12mg, including 1-12mg, such as 2-12mg of Compound (I), especially 2-4mg, 4-8mg or 8-12mg of Compound (I), for example 1, 2, 3, 4, 4 to 8, 8 to 12 mg of Compound (I). Thus for example in the above mentioned compositions, including unit doses, or treatments the Ethanedisulfonate or a pharmaceutically acceptable solvate thereof is present in an amount providing 1mg of Compound (I); the Ethanedisulfonate or a pharmaceutically acceptable solvate thereof is present in an amount providing 2mg of Compound (I); the Ethanedisulfonate or a pharmaceutically acceptable solvate thereof is present in an amount providing 3mg of Compound (I); or the Ethanedisulfonate or a pharmaceutically acceptable solvate thereof is present in an amount providing 4mg of Compound (I).

The other antidiabetic agents are suitably selected from biguanides, sulphonylureas and alpha glucosidase inhibitors. Suitable antidiabetic agents are those disclosed in WO98/57649 WO98/57634, WO98/57635, WO98/57636 WO99/03477, WO99/03476.

No adverse toxicological effects are indicated in the above mentioned treatments for the compounds of the invention.

The following Examples illustrate the invention but do not limit it in any way.

### Example 1 5-[4-[2-(N-methyl-N-(2-pyridyl)amino)ethoxy]benzyl]thiazolidine-2,4-dione 1,2-ethanedisulfonate Form I

A solution of 1,2-ethanedisulfonic acid (1.91 g) in 2-propanol (10 ml) was added to a stirred suspension of 5-[4-[2-(N-methyl-N-(2-pyridyl)amino)ethoxy]benzyl]thiazolidine-2,4-dione (3.0 g) in propan-2-ol (30 ml) at 70°C. The mixture was heated to reflux to give a clear solution and was then cooled with stirring to 21°C over approximately 1 hour. The solid was recovered by filtration, washed with propan-2-ol (10 m) and dried under vacuum at 21°C for 20 hours to give 5-[4-[2-(N-methyl-N-(2-pyridyl)amino)ethoxy]benzyl]thiazolidine-2,4-dione 1,2-ethanedisulfonate (3.79 g) as a white crystalline solid.

¹H-NMR (d6-DMSO): Consistent with 5-[4-[2-(N-methyl-N-(2-pyridyl)amino)ethoxy]benzyl]thiazolidine-2,4-dione 1,2-ethanedisulfonate (2:1).

Water content: 0.49% w/w (measured by Karl-Fischer)

### Example 2 5-[4-[2-(N-methyl-N-(2-pyridyl)amino)ethoxy]benzyl]thiazolidine-2,4-dione 1,2-ethanedisulfonate Form I

A solution of 1,2-ethanedisulfonic acid (1.91 g) in ethanol (10 ml) was added to a stirred mixture of 5-[4-[2-(N-methyl-N-(2-pyridyl)amino)ethoxy]benzyl]thiazolidine-2,4-dione (3.0 g) and ethanol (30 ml) at 60°C. The stirred mixture was heated to reflux to give initially a clear solution, followed by crystallisation to give a suspension. At this point the stirred mixture was cooled to 21°C over a period of approximately 1 hour. The product was collected by filtration, washed with ethanol (10 ml) and dried under vacuum at 21 °C for 16 hours to yield 5-[4-[2-(N-methyl-N-(2-pyridyl)amino)ethoxy]benzyl] thiazolidine-2,4-dione 1,2-ethanedisulfonate (3.55 g) as a white crystalline solid.

¹H-NNR (d6-DMSO): Consistent with 5-[4-[2-(N-methyl-N-(2-pyridyl)amino)ethoxy]benzyl]thiazolidine-2,4-dione 1,2-ethanedisulfonate (2:1).

### Example 3 5-[4-[2-(N-methyl-N-(2-pyridyl)amino)ethoxy]benzyl]thiazolidine-2,4-dione 1,2-ethanedisulfonate Form I

A solution of 1,2-ethanedisulfonic acid (1.91 g) in industrial methylated spirits (IMS) (10 ml) was added to a stirred suspension of 5-[4-[2-(N-methyl-N-(2-pyridyl)amino)ethoxy]benzyl]thiazolidine-2,4-dione (3.0 g) in IMS (30 ml) at reflux. The stirred mixture was maintained at reflux for 20 minutes to give a clear solution and was then cooled to 21°C over approximately 1 hour. The solid was recovered by filtration, washed with IMS (10 ml) and dried under vacuum for 40 hours to afford 5-[4-[2-(N-methyl-N-(2-pyridyl)amino)ethoxy]benzyl]thiazolidine-2,4-dione 1,2-ethanedisulfonate (2.49 g) as a white crystalline solid.

¹H-NMR (d6-DMSO): Consistent with 5-[4-[2-(N-methyl-N-(2-pyridyl)amino)ethoxy]benzyl]thiazolidine-2,4-dione 1,2-ethanedisulfonate (2:1).

### Characterising data for the Ethanedisulfonate Form I recorded for the product of Example 1

The infrared absorption spectrum of a mineral oil dispersion of the product was obtained using a Nicolet 710 FT-IR spectrometer at 2 cm⁻¹ resolution (Figure 1). Data were digitised at 1 cm⁻¹ intervals. Bands were observed at: 2769, 1748, 1694, 1644, 1612, 1544, 1513, 1417, 1324, 1303, 1279, 1248, 1227, 1214, 1200, 1181, 1163, 1110, 1094, 1080, 1055, 1026, 996, 929, 894, 822, 763, 737, 717, 662, 617, 604, 559, 550, 527, 518, 504 cm⁻¹.

The infrared spectrum of the solid product was recorded using Perkin-Elmer Spectrum One FT-IR spectrometer fitted with a universal ATR accessory. Bands were observed at: 2933, 2770, 1748, 1694, 1643, 1612, 1544, 1513, 1473, 1452, 1416, 1391, 1357, 1324, 1304, 1279, 1247, 1214, 1199.1181, 1161, 1111, 1095, 1056, 1025, 996, 930, 894, 822, 761, 737, 714, 668 cm⁻¹.

The Raman spectrum of the product (Figure 2) was recorded with the sample in a glass vial using a Perkin-Elmer 2000R FT-Raman spectrometer, at 4 cm⁻¹ resolution with excitation from a Nd:YAG laser (1064 nm) with a power output of 400mW. Bands were observed at: 3068, 2936, 1748, 1695, 1613, 1545, 1448, 1415, 1391, 1323, 1299, 1272, 1209, 1183, 1053, 979, 893, 823, 775, 740, 660, 637, 604, 527, 469, 434, 394, 332, 293 cm⁻¹.

The X-Ray Powder Diffractogram pattern of the product (**Figure 3**) was recorded using the following acquisition conditions: Tube anode: Cu, Generator tension: 40 kV, Generator current: 40 mA, Start angle: 2.0 °2θ, End angle: 35.0 °2θ, Step size: 0.02 °2θ, Time per step: 2.5 seconds. Characteristic XRPD angles and relative intensities are recorded in Table 1.

**Table 1**

| **Angle** | **Rel. Intensity** |
|---|---|
| **2-Theta** ° | % |
| 6.4 | 9.3 |
| 9.2 | 4.9 |
| 11.0 | 9.1 |
| 12. | 2.2 |
| 12.8 | 10 |
| 13.3 | 7.1 |
| 14.0 | 9.2 |
| 14.5 | 2.7 |
| 15.2 | 12 |
| 15.5 | 6.3 |
| 17.2 | 53.2 |
| 18.0 | 26.6 |
| 18.4 | 8.1 |
| 18.9 | 5.6 |
| 19.5 | 17.1 |
| 20.1 | 5.3 |
| 21.0 | 16.8 |
| 21.5 | 100 |
| 21.8 | 23.2 |
| 22.2 | 29.3 |
| 22.7 | 7.9 |
| 23.3 | 9.5 |
| 24.1 | 29.3 |
| 24.7 | 11.8 |
| 25.2 | 28 |
| 25.9 | 18.3 |
| 26.4 | 8.3 |
| 26.8 | 14.3 |
| 27.6 | 5.9 |
| 27.9 | 13.2 |
| 28.4 | 8.1 |
| 29.0 | 6.7 |
| 29.7 | 10.3 |
| 30.3 | 8.2 |
| 30.7 | 8.4 |
| 31.0 | 8 |
| 31.4 | 7.9 |
| 31.9 | 5.2 |
| 32.3 | 7.4 |
| 33.0 | 6.1 |
| 33.4 | 11 |
| 33.9 | 6.4 |
| 34.8 | 7.3 |

The solid-state NMR spectrum of the product (Figure 4) was recorded on a Bruker AMX360 instrument operating at 90.55 MHz: The solid was packed into a 4 mm zirconia MAS rotor fitted with a Kel-F cap and rotor spun at ca.10 kHz. The ¹³C MAS spectrum was acquired by cross-polarisation from Hartmann-Hahn matched protons (CP contact time 3 ms, repetition time 15 s) and protons were decoupled during acquisition using a two-pulse phase modulated (TPPM) composite sequence. Chemical shifts were externally referenced to the carboxylate signal of glycine at 176.4 ppm relative to TMS and were observed at: 33.4, 35.9, 38.4, 40.4, 42.6, 47.4, 50.8, 53.3, 54.6, 64.3, 69.1, 110,1, 113.6, 116.2, 119.7, 125.9,128.0, 130.2, 131.6, 136.2, 138.1, 144.2, 146.2, 147.2, 151.3, 152.7, 157.4, 158.9, 171.8, 174.5,176.5 ppm.

### Properties of the Ethanedisulfonate Form I

**Solubility of the Ethanedisulfonate Form I, recorded for the product of Example 2** The solubility of the material was determined by adding water in aliquots from 0.25 to 1ml to approximately 30 mg of drug substance until the powder had dissolved Solubility: 4.7 mg/ml.

**Melting Range of the Ethanedisulfonate Form I, recorded for the product of Example 1** The melting range of the Ethanedisulfonate Form I was determined according to the method described in the U.S. Pharmacopoeia, USP 23, 1995, <741> "Melting range or temperature, Procedure for Class Ia", using a Buchi 545 melting point instrument.
Melting range: 177.0-177.6°C

### Example 4 5-[4-[2-(N-methyl-N-(2-pyridyl)amino)ethoxy]benzyl]thiazolidine-2,4-dione 1,2-ethanedisulfonate Form II

A stirred suspension of 5-[4-[2-(N-methyl-N-(2-pyridyl)amino)ethoxy]benzyl] thiazolidine-2,4-dione (3.0 g) in ethanol (30 ml) was heated to reflux before a solution of 1,2-ethanedisulfonic acid (1.91 g) in ethanol (10 ml) was added. The stirred reaction mixture was maintained at reflux until a clear solution was observed, and was then cooled to 21°C over approximately 1 hour. The solid was collected by filtration, washed with ethanol (10 ml) and dried under vacuum for 16 hours to give 5-[4-[2-(N-methyl-N-(2-pyridyl)amino)ethoxy]benzyl]thiazolidine-2,4-dione 1,2-ethanedisulfonate (3.69 g) as a white crystalline solid.

¹H-NMR (d6-DMSO): Consistent with 2:1 5-[4-[2-(N-methyl-N-(2-pyridyl)amino)ethoxy]benzyl]thiazolidine-2,4-dione 1,2-ethanedisulfonate containing 0.6% w/w of ethanol.

Water content: 0.24% w/w (measured by Karl-Fischer)

### Example 5 5-[4-[2-(N-methyl-N-(2-pyridyl)amino)ethoxy]benzyl]thiazolidine-2,4-dione 1,2-ethanedisulfonate Form II

A stirred solution of 5-[4-[2-(N-methyl-N-(2-pyridyl)amino)ethoxy]benzyl]thiazolidine-2,4-dione (3.0 g) in tetrahydrofuran (90 ml) was heated to reflux before a solution of 1,2-ethanedisulfonic acid (1.91 g) in tetrahydrofuran (10 ml) was added, resulting in the formation of a precipitate. The stirred mixture was cooled to 21°C over approximately 1 hour. The solid was collected by filtration, washed with tetrahydrofuran (10 ml) and dried for 40 hours under vacuum to afford 2:1 5-[4-[2-(N-methyl-N-(2-pyridyl)amino)ethoxy]benzyl]thiazolidine-2,4-dione 1,2-ethanedisulfonate (3.56 g) as a white crystalline solid.

¹H-NMR (d6-DMSO): Consistent with 2:1 5-[4-[2-(N-methyl-N-(2-pyridyl)amino)ethoxy]benzyl]thiazolidine-2,4-dione 1,2-ethanedisulfonate.

### Example 6 5-[4-[2-(N-methyl-N-(2-pyridyl)amino)ethoxy]benzyl]thiazolidine-2,4-dione 1,2-ethanedisulfonate Form II

A solution of 1,2-ethanedisulfonic acid (1.68 g) in acetonitrile (10 ml) and water (10 ml) was added to a stirred suspension of 5-[4-[2-(N-methyl-N-(2-pyridyl)amino)ethoxy]benzyl]thiazolidine-2,4-dione (3.0 g) in acetonitrile (100 ml) at 21°C. The reaction mixture was heated to 85°C for 1 hour, cooled to 21°C over 30 minutes, then stirred for 4 hours at 21 °C. The solid was collected by filtration, washed with acetonitrile (30 ml) and dried under vacuum to give 5-[4-[2-(N-methyl-N-(2-pyridyl)amino)ethoxy]benzyl]thiazolidine-2,4-dione 1,2-ethanedisulfonate (1.84 g) as a white crystalline solid.

¹H-NMR (d6-DMSO): Consistent with 2:1 5-[4-[2-(N-methyl-N-(2-pyridyl)amino)ethoxy]benzyl]thiazolidine-2,4-dione 1,2-ethanedisulfonate.

### Example 7 5-[4-[2-(N-methyl-N-(2-pyridyl)amino)ethoxy]benzyl]thiazolidine-2,4-dione 1,2-ethanedisulfonate Form II

A solution of ethanedisulfonic acid (12.73 g) dissolved in ethanol (70 mL) was added to a stirred suspension of 5-[4-[2-(N-methyl-N-(2-pyridyl)amino)ethoxy]benzyl] thiazolidine-2,4-dione (20.0 g) in ethanol (200 mL) at reflux. The mixture was maintained at reflux for 10 minutes and then cooled to 21°C over approximately 1 hour. The solid was collected by filtration, washed with ethanol (2 x 20 mL) and dried for 16 hours under vacuum to afford 5-[4-[2-(N-methyl-N-(2-pyridyl)amino)ethoxy]benzyl] thiazolidine-2,4-dione 1,2-ethanedisulfonate (24.67 g) as a white crystalline solid.

¹H-NMR (d6-DMSO): Consistent with 2:1 5-[4-[2-(N-methyl-N-(2-pyridyl)amino)ethoxy]benzyl]thiazolidine-2,4-dione 1,2-ethanedisulfonate containing 0.2%w/w ethanol.

Water content: 0.05% w/w (measured by Karl-Fischer)

### Example 8 5-[4-[2-(N-methyl-N-(2-pyridyl)amino)ethoxy]benzyl]thiazolidine-2,4-dione 1,2-ethanedisulfonate Form II

A stirred suspension of 5-[4-[2-(N-methyl-N-(2-pyridyl)amino)ethoxy]benzyl] thiazolidine-2,4-dione (3.0 g) in propan-2-ol (30 mL) was heated to reflux before a solution of ethanedisulfonic acid (1.91 g) in water (5 mL) was added. The solution was maintained at reflux for 15 minutes at which time the solution was clear. The solution was cooled to 50°C and seeded with the product of Example 2 (0.30 g). The mixture was maintained at 50°C for 30 minutes and then cooled to 21°C. The solid was collected by filtration to afford 5-[4-[2-(N-methyl-N-(2-pyridyl)amino)ethoxy]benzyl]thiazolidine-2,4-dione 1,2-ethanedisulfonate (3.85 g) as a white crystalline solid.

### Example 9 5-[4-[2-(N-methyl-N-(2-pyridyl)amino)ethoxy]benzyl]thiazolidine-2,4-dione 1,2-ethanedisulfonate Form II

A solution of ethanedisulfonic acid (1.91 g) in water (7 mL) was added to a stirred suspension of 5-[4-[2-(N-methyl-N-(2-pyridyl)amino)ethoxy]benzyl] thiazolidine-2,4-dione (3.0 g) in ethyl acetate (30 mL) at reflux. The mixture was maintained at reflux until a clear solution was formed. The solution was cooled to 45°C and seeded with the product of Example 2 (0.12 g). The mixture was then cooled to 21°C, before the solid was collected by filtration and dried on the filter for 2 hours to give 5-[4-[2-(N-methyl-N-(2-pyridyl)amino)ethoxy]benzyl]thiazolidine-2,4-dione 1,2-ethanedisulfonate (2.90 g) as a white crystalline solid.

### Characterising data for the Ethanedisulfonate Form II recorded for the product of Example 4

The infrared absorption spectrum of a mineral oil dispersion of the product was obtained using a Nicolet 710 FT-IR spectrometer at 2 cm⁻¹ resolution (Figure 5). Data were digitised at 1 cm⁻¹ intervals. Bands were observed at: 3332, 2765, 1750, 1695, 1644, 1616, 1582, 1546, 1513, 1413, 1332, 1315, 1274, 1250, 1228, 1207, 1183, 1162, 1114, 1100, 1077, 1052, 1022, 993, 916, 904, 854, 830, 818, 770, 759, 732, 717, 684, 654, 604, 547, 535, 513 cm⁻¹.

The infrared spectrum of the solid product was recorded using Perkin-Elmer Spectrum One FT-IR spectrometer fitted with a universal ATR accessory. Bands were observed at: 3333, 3116, 2906, 2768, 1750, 1693, 1644, 1615, 1584, 1547, 1514, 1473, 1448, 1413, 1370, 1332, 1315, 1274, 1249, 1227, 1207, 1160, 1114,1100, 1077, 1052, 1021, 993, 933, 916, 904, 854, 830, 818, 759, 731, 716, 682 cm⁻¹.

The Raman spectrum of the product (Figure 6) was recorded with the sample in a glass vial using a Perkin-Elmer 2000R FT-Raman spectrometer, at 4 cm-1 resolution with excitation from a Nd:YAG laser (1064 nm) with a power output of 400mW. Bands were observed at: 3099, 3055, 2965, 2917, 1747, 1706, 1645, 1612, 1585, 1547, 1462, 1438, 1382, 1327, 1305, 1278, 1257, 1238, 1210, 1179, 1150, 1096, 1035, 985, 934, 901, 844, 826, 779, 742, 664, 639, 617, 604, 558, 540, 472, 441, 423, 401, 347, 288 cm-1,.

The X-Ray Powder Diffractogram pattern of the product (**Figure 7**) was recorded using the following acquisition conditions: Tube anode: Cu, Generator tension: 40 kV; Generator current: 40 mA, Start angle: 2.0 °2θ, End angle: 35.0 °2θ, Step size: 0.02 °2θ , Time per step: 2.5 seconds. Characteristic XRPD angles and relative intensities are recorded in Table 2.

**Table 2**

| **Angle** | **Rel. Intensity** |
|---|---|
| **2-Theta °** | **%** |
| 6.2 | 11.7 |
| 11.4 | 3.3 |
| 11.7 | 4.4 |
| 12.6 | 25.9 |
| 14.4 | 58.3 |
| 14.8 | 12.7 |
| 15.1 | 4.9 |
| 16.0 | 3.8 |
| 17.4 | 18.3 |
| 18.7 | 9.9 |
| 19.3 | 23.8 |
| 19.9 | 73.1 |
| 21.5 | 82.2 |
| 21.7 | 100 |
| 22.3 | 9.9 |
| 23.1 | 9.8 |
| 23.4 | 16.4 |
| 23.7 | 67.7 |
| 24.4 | 27.9 |
| 24.8 | 15.6 |
| 26.0 | 14.3 |
| 26.8 | 14 |
| 27.3 | 16.2 |
| 27.9 | 10.4 |
| 29.1 | 10.3 |
| 29.9 | 8.6 |
| 30.9 | 14.7 |
| 32.3 | 27.8 |
| 32.6 | 29.4 |
| 33.5 | 10.8 |

The solid-state NMR spectrum of the product (**Figure 8**) was recorded on a Bruker AMX360 instrument operating at 90.55 MHz: The solid was packed into a 4 mm zirconia MAS rotor fitted with a Kel-F cap and rotor spun at ca.10 kHz. The ¹³C MAS spectrum was acquired by cross-polarisation from Hartmann-Hahn matched protons (CP contact time 3 ms, repetition time 15 s) and protons were decoupled during acquisition using a two-pulse phase modulated (TPPM) composite sequence. Chemical shifts were externally referenced to the carboxylate signal of glycine at 176.4 ppm relative to TMS and were observed at: 33.7, 36.1, 47.5, 50.9, 52.9, 64.6, 110.0, 116.5, 119.8, 126.1, 128.4, 131.9, 138.4, 144.5, 153.2, 157.6, 171.8, 174.8, 176.4 ppm.

### Properties of the Ethanedisulfonate Form II, recorded for the product of Example 7

### Solid State Stability of the Ethanedisulfonate Form II

The solid state stability of the drug substance was determined by storing approximately 1.0 g of the material in a glass bottle at a) 40°C / 75% Relative Humidity (RH), open exposure, for 1 month and b) at 50°C, closed, for 1 month. The material was assayed by HPLC for final content and degradation products in both cases.
a) 40°C / 75% RH: No significant degradation observed (HPLC assay 98% initial).
b) 50°C: No significant degradation observed (HPLC assay 99% initial).

### Solubility of the Ethanedisulfonate Form II

The solubility of the material was determined by adding water in aliquots from 1 to 1000ml to approximately 100mg of drug substance until the powder had dissolved. The visual solubility was confirmed by an HPLC assay of a saturated solution. Solubility: 4.7 mg/ml.

### Flow Properties of the Ethanedisulfonate Form

The ratio between the bulk density and the tapped bulk density (Hausner Ratio) of the Ethanedisulfonate was determined using standard methods ("Pharmaceutics - The Science of Dosage Form Design", editor M. Aulton, 1988, published by:Churchill Livingstone).
Hausner Ratio: 1.1

### Tₒₙₛₑₜ of the Ethanedisulfonate Form II

The Tₒₙₛₑₜ of the drug substance was determined by Differential Scanning Calorimetry using a Perkin-Elmer DSC7 apparatus.
Tₒₙₛₑₜ (10°C/minute, closed pan): 197 °C

### Melting Range of the Ethanedisulfonate Form II

The melting range of the Ethanedisulfonate Form II was determined according to the method described in the U.S. Pharmacopoeia, USP 23, 1995, <741> "Melting range or temperature, Procedure for Class Ia", using a Buchi 545 melting point instrument.
Melting range: 197.8-199.0°C

## Claims

1. A salt of S-[4-[2-(N-methyl-N-(2-pyridyl)amino)ethoxy]benzyl]thiazolidine-2,4-dione and 1,2-ethanedisulfonic acid or a solvate thereof.

2. A salt according to claim 1, being 5-[4-[2-(N-methyl-N-(2-pyridyl)amino)ethoxy]benzyl]thiazolidine-2,4-dione (2:1) 1,2-ethanedisulfonate or a solvate thereof.

3. A 5-[4-[2-(N-methyl-N-(2-pyridyl)amino)ethoxy]benzyl]thiazolidine-2,4-dione 1,2-ethanedisulfonate according to claim 1 or 2, **characterised in that** it provides:
(i) an infrared spectrum containing peaks at about 1303, 1214, 1026 and 763 cm⁻¹; and/or
(ii) a Raman spectrum containing peaks at about 1415, 1323, 1053, and 293 cm⁻¹;
(iii) an X-ray powder diffraction (XRPD) pattern containing peaks at about 9.2, 11.0, 13.3, 15.5, 17.2, and 18.0 degrees 2θ.

4. A 5-[4-[2-(N-methyl-N-(2-pyridyl)amino)ethoxy]benzyl]thiazolidine-2,4-dione (2:1) 1,2-ethanedisulfonate salt, according to any one of claims 1 to 3, **characterised in that** it provides an infrared spectrum substantially in accordance with Figure 1.

5. A 5-[4-[2-(N-methyl-N-(2-pyridyl)amino)ethoxy]benzyl]thiazolidine-2,4-dione (2:1) 1,2-ethanedisulfonate salt, according to any one of claims 1 to 4, **characterised in that** it provides a Raman spectrum substantially in accordance with Figure 2.

6. A 5-[4-[2-(N-methyl-N-(2-pyridyl)amino)ethoxy]benzyl]thiazolidine-2,4-dione (2:1) 1,2-ethanedisulfonate salt, according to any one of claims 1 to 5, **characterised in that** it provides an X-Ray powder diffraction pattern (XRPD) substantially in accordance with Table 1 or Figure 3.

7. A 5-[4-[2-(N-methyl-N-(2-pyridyl)amino)ethoxy]benzyl]thiazolidine-2,4-dione (2:1) 1,2-ethanedisulfonate salt, according to any one of claims 1 to 6, **characterised in that** it provides a Solid State ¹³C NMR spectrum substantially in accordance with Figure 4.

8. A 5-[4-[2-(N-methyl-N-(2-pyridyl)amino)ethoxy]benzyl]thiazolidine-2,4-dione (2:1) 1,2-ethanedisulfonate salt, according to any one of claims 1 or 7, **characterised in that** it provides:
(i) an infrared spectrum substantially in accordance with Figure 1; and
(ii) a Raman spectrum substantially in accordance with Figure 2; and
(iii) an X-Ray powder diffraction pattern (XRPD) substantially in accordance with Table 1 or Figure 3; and
(iv) a Solid State ¹³C NMR spectrum substantially in accordance with Figure 4.

9. A 5-[4-[2-(N-methyl-N-(2-pyridyl)amino)ethoxy]benzyl]thiazolidine-2,4-dione 1,2-ethanedisulfonate Form II salt, according to claim 1 or 2 **characterised in that** it provides
(i) an infrared spectrum containing peaks at about 1315, 1274, 1022, 904 and 513 cm⁻¹; and/or
(ii) a Raman spectrum containing peaks at about 1706, 1645, 1382, 1327 and 901 cm⁻¹; and/or
(iii) an X-ray powder diffraction (XRPD) pattern containing peaks at about 12.6, 14.8, 19.9 and 23.7 degrees 2θ.

10. A 5-[4-[2-(N-methyl-N-(2-pyridyl)amino)ethoxy]benzyl]thiazolidine-2,4-dione (2:1) 1,2-ethanedisulfonate salt, according to claims 1, 2 or 9 **characterised in that** it provides an infrared spectrum substantially in accordance with Figure 5.

11. A 5-[4-[2-(N-methyl-N-(2-pyridyl)amino)ethoxy]benzyl]thiazolidine-2,4-dione (2:1) 1,2-ethanedisulfonate salt, according to claim 10, **characterised in that** it provides a Raman spectrum substantially in accordance with Figure 6.

12. A 5-[4-[2-(N-methyl-N-(2 pyridyl)amino)ethoxy]benzyl]thiazolidine-2,4-dione (2:1) 1,2-ethanedisulfonate salt, according to claim 10 or 11, **characterised in that** it provides an X-Ray powder diffraction pattern (XRPD) substantially in accordance with Table 2 or Figure 7.

13. A 5-[4-[2-(N-methyl-N-(2 pyridyl)amino)ethoxy]benzyl]thiazolidine-2,4-dione (2:1) 1,2-ethanedisulfonate salt, according to any one of claims 10 to 12, **characterised in that** it provides a Solid State ¹³C NMR spectrum substantially in accordance with Figure 8.

14. A 5-[4-[2-(N-methyl-N-(2-pyridyl)amino)ethoxy]benzyl]thiazolidine-2,4-dione (2:1) 1,2-ethanedisulfonate salt, according to any one of claims 10 to 13, **characterised in that** it provides:
(i) an infrared spectrum substantially in accordance with Figure 5; and
(ii) a Raman spectrum substantially in accordance with Figure 6; and
(iii) an X-Ray powder diffraction pattern (XRPD) substantially in accordance with Table 2 or Figure 7; and
(iv) a Solid State ¹³C NMR spectrum substantially in accordance with Figure 8.

15. A salt according to claims 1, being 5-[4-[2-(N-methyl-N-(2-pyridyl)amino)ethoxy]benzyl]thiazolidine-2,4-dione 1,2-ethanedisulfonate, being a 1:1 salt.

16. A compound according to any one of claims 1 to 15, or a solvate thereof, in isolated form.

17. A compound according to any one of claims 1 to 15, or a solvate thereof, in substantially pure form.

18. A compound according to any one of claims 1 to 15, or a solvate thereof, in crystalline form.

19. A process for preparing a compound according to any one of claims 1 to 15, or a solvate thereof, **characterised in that** 5-[4-[2-(N-methyl-N-(2-pyridyl)amino)ethoxy]benzyl]thiazolidine-2,4-dione (Compound (I)) or a salt thereof, preferably dispersed or dissolved in a suitable solvent, is reacted with a suitable source of ethanedisulfonate ion; and the ethanedisulfonate salt or a solvate thereof is recovered.

20. A salt of 5-[4-[2-(N-methyl-N-(2-pyridyl)amino)ethoxy]benzyl]thiazolidine-2,4-dione and 1,2-ethanedisulfonic acid or a pharmaceutically acceptable solvate thereof, according to claim 1, for use as an active therapeutic substance.

21. A salt of 5-[4-[2-(N-methyl-N-(2-pyridyl)amino)ethoxy]benzyl]thiazolidine-2,4-dione and 1,2-ethanedisulfonic acid or a pharmaceutically acceptable solvate thereof, according to claim 1, for use in the treatment and/or prophylaxis of diabetes mellitus, conditions associated with diabetes mellitus and certain complications thereof.

22. A use of a salt of 5-[4-[2-(N-methyl-N-(2-pyridyl)amino)ethoxy]benzyl]thiazolidine-2,4-dione and 1,2-ethanedisulfonic acid or a pharmaceutically acceptable solvate thereof, according to claim 1, for the manufacture of a medicament for the treatment and/or prophylaxis of diabetes mellitus, conditions associated with diabetes mellitus and certain complications thereof.

23. A use as claimed in claim 22, wherein the medicament comprises, in combination, one or more other anti-diabetic agents.

## Patentansprüche

1. Salz von 5-[4-[2-(N-Methyl-N-(2-pyridyl)amino)ethoxy]benzyl]thiazolidin-2,4-dion und 1,2-Ethandisulfonsäure oder ein Solvat davon.

2. Salz gemäß Anspruch 1, nämlich 5-[4-[2-(N-Methyl-N-(2-pyridyl)amino)ethoxy]benzyl]thiazolidin-2,4-dion-(2:1)-1,2-ethandisulfonat oder ein Solvat davon.

3. 5-[4-[2-(N-Methyl-N-(2-pyridyl)amino)ethoxy]benzyl]thiazolidin-2,4-dion-1,2-ethandisulfonat gemäß Anspruch 1 oder 2, **dadurch gekennzeichnet, dass** es:
(i) ein Infrarotspektrum, welches Peaks bei etwa 1303, 1214, 1026 und 763 cm⁻¹ enthält;
und/oder
(ii) ein Ramanspektrum, welches Peaks bei etwa 1415, 1323, 1053 und 293 cm⁻¹ enthält;
(iii) ein Röntgenpul verbeugungs-(XRPD)-muster, welches Peaks bei etwa 9,2, 11,0, 13,3, 15,5, 17,2 und 18,0 Grad 20 enthalt,
liefert.

4. 5-[4-[2-(N-Methyl-N-(2-pyridyl)amino)ethoxy]benzyl]thiaxolidin-2,4-dion-(2.1)-1,2-ethandisulfonatsalz gemäß einem der Ansprüche 1 bis 3, **dadurch gekennzeichnet, dass** es ein Infrarotspektrum im wesentlichen gemäß Figur 1 liefert.

5. 5-[4-[2-(N-Methyl-N-(2-pyridyl)amino)ethoxy]benzyl]thiazolidin-2,4-dion-(2:1)-1,2-ethandisulfonatsalz gemäß einem der Ansprüche 1 bis 4, **dadurch gekennzeichnet, dass** es ein:Ramanspektrum im wesentlichen gemäß Figur 2 liefert.

6. 5-[4-[2-(N-Methyl-N-(2-pyridyl)amino)ethoxy]benzyl]thiazolidin-2,4-dion-(2:1)-1,2-ethandisulfonatsalz gemäß einem der Ansprüche 1 bis 5, **dadurch gekennzeichnet, dass** es ein Röntgenpulverbeugungs-(XRPD)-muster im wesentlichen gemäß Tabelle 1 oder Figur 3 liefert.

7. 5-[4-[2-(N-Methyl-N-(2-pyridyl)amino)ethoxy]benzyl]thiazolidin-2,4-dion-(2:1)-1,2-ethandisulfonatsalz gemäß einem der Ansprüche 1 bis 6, **dadurch gekennzeichnet, dass** es ein Festkörper⁻¹³C NMR-Spektrum im wesentlichen gemäß Figur 4 liefert.

8. 5-[4-[2-(N-Methyl-N-(2-pyridyl)amino)ethoxy]benzyl]thiazolidin-2,4-dion-(2:1)-1,2-ethandisulfonatsalz gemäß einem der Ansprüche 1 oder 7, **dadurch gekennzeichnet, dass** es:
(i) ein Infrarotspektrum im wesentlichen gemäß Figur 1; und
(ii) ein Ramanspektrum im wesentlichen gemäß Figur 2; und
(iii) ein Röntgenpulverbeugungs-(XRPD)-muster im wesentlichen gemäß Tabelle 1 oder Figur 3; und
(iv) ein Festkörper-¹³C NMR-Spektrum im wesentlichen gemäß Figur 4,
liefert.

9. 5-[4-[2-(N-Methyl-N-(2-pyridyl)amino)ethoxy]benzyl]thiazolidin-2,4-dion-1,2-ethandisulfonatsalz der Form II gemäß Anspruch 1 oder 2, **dadurch gekennzeichnet, dass** es:
(i) ein Infrarotspektrum, welches Peaks bei etwa 1315, 1274, 1022, 904 und 513 cm⁻¹ enthält;
und/oder
(ii) ein Ramanspektrum, welches Peaks bei etwa 1706, 1645, 1382, 1327 und 901 cm⁻¹ enthält; und/oder
(iii) ein Röntgenpulverbeugungs-(XRPD)-muster, welches Peaks bei etwa 12,6, 14,8, 19,9 und 23,7 Grad 2Θ enthält,
liefert.

10. 5-[4-[2-(N-Methyl-N-(2-pyridyl)amino)ethoxy]benzyl]thiazolidin-2,4-dion-(2:1)-1,2-ethandisulfonatsalz gemäß den Ansprüchen 1, 2 oder 9, **dadurch gekennzeichnet, dass** es ein Infrarotspektrum im wesentlichen gemäß Figur 5 liefert.

11. 5-[4-[2-(N-Methyl-N'-(2-pyridyl)amino)ethoxy]benzyl]thiazolidin-2,4-dion-(2:1)-1,2-ethandisulfonatsalz gemäß Anspruch 10, **dadurch gekennzeichnet, dass** es ein Ramanspektrum im wesentlichen gemäß Figur 6 liefert.

12. 5-[4-[2-(N-Methyl-N-(2-pyridyl)amino)ethoxy]benzyl]thiazolidin-2,4-dion-(2:1)-1,2-ethandisulfonatsalz gemäß Anspruch 10 oder 11, **dadurch gekennzeichnet, dass** es ein Röntgenpulverbeugungs-(XRPD)-muster im wesentlichen gemäß Tabelle 2 oder Figur 7 liefert.

13. 5-[4-[2-(N-Methyl-N-(2-pyridyl)amino)ethoxy]benzy]thiaxolidin-2,4-dion-(2:1)-1,2-ethandisulfonatsalz gemäß einem der Ansprüche 10 bis 12, **dadurch gekennzeichnet, dass** es ein Festkörper-¹³C NMR-Spektrum im wesentlichen gemäß Figur 8 liefert.

14. 5-[4-[2-(N-Methyl-N-(2-pyridyl)amino)ethoxy]benzyl]thiazolidin-2,4-dion-(2:1)-1,2-ethandisulfonatsalz gemäß einem der Ansprüche 10 bis 13, **dadurch gekennzeichnet, dass** es:
(i) ein Infrarotspektrum im wesentlichen gemäß Figur 5; und
(ii) ein Ramanspektrum im wesentlichen gemäß Figur 6; und
(iii) ein Röntgenpulverbeugungs-(XRPD)-muster im wesentlichen gemäß Tabelle 2 oder Figur 7; und
(iv) ein Festkörper-¹³C NMR-Spektrum im wesentlichen gemäß Figur 8, liefert.

15. Salz gemäß Anspruch 1, nämlich 5-[4-[2-(N-Methyl-N-(2-pyridyl)amino)-ethoxy]benzyl]thiazolidin-2,4-dion-1,2-ethandisulfonatsalz, wobei es ein 1:1 1 Salz ist.

16. Verbindung gemäß einem der Ansprüche 1 bis 15, oder ein Solvat davon, in isolierter Form.

17. Verbindung gemäß einem der Ansprüche 1 bis 15, oder ein Solvat davon, in im wesentlichen reiner Form.

18. Verbindung gemäß einem der Ansprüche 1 bis 15, oder ein Solvat davon, in kristalliner Form.

19. Verfahren zur Herstellung einer Verbindung gemäß einem der Ansprüche 1 bis 15 oder eines Solvats davon, **dadurch gekennzeichnet, dass** 5-[4-[2-(N-Methyl-N-(2-pyridyl)amino)ethoxy]benzyl]thiazolidin-2,4-dion (Verbindung (I)) oder ein Salz davon, vorzugsweise in einem geeigneten Lösungsmittel dispergiert oder aufgelöst, mit einer geeigneten Ethandisulfonationquelle umgesetzt wird und das Ethandisulfonatsalz oder ein Solvat davon gewonnen wird.

20. Salz von 5-[4-[2-(N-Methyl-N-(2-pyridyl)amino)ethoxy]benzyl]thiazolidin-2,4-dion und 1,2-Ethandisulfonsäure oder eines pharmazeutisch verträglichen Solvats davon gemäß Anspruch 1, zur Verwendung als therapeutischer Wirkstoff.

21. Salz von 5-[4-[2-(N-Methyl-N-(2-pyridyl)amino)ethoxy]benzyl]thiazolidin-2,4-dion und 1,2 Ethandisulfonsäure oder eines pharmazeutisch verträglichen Solvats davon gemäß Anspruch 1, zur Verwendung in der Behandlung und/oder Prophylaxe von Diabetes mellitus, mit Diabetes mellitus verbundenen Zuständen und gewissen Komplikationen **dadurch**.

22. Verwendung eines Salzes von 5-[4-[2-(N-Methyl-N-(2-pyridyl)amino)ethoxy]-benzyl]thiazolidin-2,4-dion und 1,2-Ethandisulfonsäure oder eines pharmazeutisch verträglichen Solvats davon gemäß Anspruch 1, zur Herstellung eines Medikaments zur Behandlung und/oder Prophylaxe von Diabetes mellitus, mit Diabetes mellitus verbundenen Zuständen und gewissen Komplikationen **dadurch**.

23. Verwendung gemäß Anspruch 22, wobei das Medikament ein oder mehrere Anti-Diabetes-Mittel in Kombination umfasst.

## Revendications

1. Sel de 5-[4-[2-(N-méthyl-N-(2-pyridyl)amino)éthoxy]benzyl] thiazolidine-2,4-dione et d'acide 1,2-éthanedisulfonique, ou un de ses produits de solvatation.

2. Sel suivant la revendication 1, qui est le 1,2-éthanedisulfonate de 5-[4-[2-(N-méthyl-N-(2-pyridyl)-amino)éthoxy]benzyl]thiazolidine-2,4-dione (2:1), ou un de ses produits de solvatation.

3. 1,2-éthanedisulfonate de 5-[4-[2-(N-méthyl-N-(2-pyridyl)amino)éthoxy]benzyl]thiazolidine-2,4-dione suivant la revendication 1 ou 2, **caractérisé en ce qu'**il présente :
(i) un spectre infrarouge contenant des pics à environ 1303, 1214, 1026 et 763 cm⁻¹ ; et/ou
(ii) un spectre Raman contenant des pics à environ 1415, 1323, 1053 et 293 cm⁻¹;
(iii) un diagramme de diffraction des rayons X sur poudre (XRPD) contenant des pics à environ 9,2, 11,0, 13,3, 15,5, 17,2 et 18,0 degrés 2θ.

4. Sel 1,2-éthanedisulfonate de 5-[4-[2-(N-méthyl-N-(2-pyridyl)amino)éthoxy]benzyl]thiazolidine-2,4-dione (2:1) suivant l'une quelconque des revendications 1 à 3, **caractérisé en ce qu'**il présente un spectre infrarouge essentiellement suivant la figure 1.

5. Sel 1,2-éthanedisulfonate de 5-[4-[2-(N-méthyl-N-(2-pyridyl)amino)éthoxy]benzyl]thiazolidine-2,4-dione (2:1) suivant l'une quelconque des revendications 1 à 4, **caractérisé en ce qu'**il présente un spectre Raman essentiellement suivant la figure 2.

6. Sel 1,2-éthanedisulfonate de 5-[4-[2-(N-méthyl-N-(2-pyridyl)amino)éthoxy]benzyl]thiazolidine-2,4-dione (2:1) suivant l'une quelconque des revendications 1 à 5, **caractérisé en ce qu'**il présente un diagramme de diffraction des rayons X sur poudre (XRPD) essentiellement suivant le tableau 1 ou la figure 3.

7. Sel 1,2-éthanedisulfonate de 5-[4-[2-(N-méthyl-N-(2-pyridyl)amino)éthoxy]benzyl]thiazolidine-2,4-dione (2:1) suivant l'une quelconque des revendications 1 à 6, **caractérisé en ce qu'**il présente un spectre de ¹³C-RMN à l'état solide essentiellement suivant la figure 4.

8. Sel 1,2-éthanedisulfonate de 5-[4-[2-(N-méthyl-N-(2-pyridyl)amino)éthoxy]benzyl]thiazolidine-2,4-dione (2:1) suivant l'une quelconque des revendications 1 ou 7, **caractérisé en ce qu'**il présente :
(i) un spectre infrarouge essentiellement suivant la figure 1 ; et
(ii) un spectre Raman essentiellement suivant la figure 2 ; et
(iii) un diagramme de diffraction des rayons X sur poudre (XRPD) essentiellement suivant le tableau 1 ou la figure 3 ; et
(iv) un spectre de ¹³C-RMN à l'état solide essentiellement suivant la figure 4.

9. Forme II du sel 1,2-éthanedisulfonate de 5-[4-[2-(N-méthyl-N-(2-pyridyl)amino)éthoxy]benzyl]thiazolidine-2,4-dione suivant la revendication 1 ou 2, **caractérisé en ce qu'**il présente :
(i) un spectre infrarouge contenant des pics à environ 1315, 1274, 1022, 904 et 513 cm⁻¹ ; et/ou
(ii) un spectre Raman contenant des pics à environ 1706, 1645, 1382, 1327 et 901 cm⁻¹; et/ou
(iii) un diagramme de diffraction des rayons X sur poudre (XRPD) contenant des pics à environ 12,6, 14,8, 19,9 et 23,7 degrés 2θ.

10. Sel 1,2-éthanedisulfonate de 5-[4-[2-(N-méthyl-N-(2-pyridyl) amino) éthoxy]benzyl] thiazolidine-2, 4-dione (2:1) suivant la revendication 1, 2 ou 9, **caractérisé en ce qu'**il présente un spectre infrarouge essentiellement suivant la figure 5.

11. Sel 1,2-éthanedisulfonate de 5-[4-[2-(N-méthyl-N-(2-pyridyl)amino)éthoxy]benzyl]thiazolidine-2,4-dione (2:1) suivant la revendication 10, **caractérisé en ce qu'**il présente un spectre Raman essentiellement suivant la figure 6.

12. Sel 1,2-éthanedisulfonate de 5-[4-[2-(N-méthyl-N-(2-pyridyl)amino)éthoxy]benzyl]thiazolidine-2,4-dione (2:1) suivant la revendication 10 ou 11, **caractérisé en ce qu'**il présente un diagramme de diffraction des rayons X sur poudre (XRPD) essentiellement suivant le tableau 2 ou la figure 7.

13. Sel 1,2-éthanedisulfonate de 5-[4-[2-(N-méthyl-N-(2-pyridyl)amino) éthoxy] benzyl] thiazolidine-2, 4-dione (2:1) suivant l'une quelconque des revendications 10 à 12, **caractérisé en ce qu'**il présente un spectre de ¹³C-RMN à l'état solide essentiellement suivant la figure 8.

14. Sel 1,2-éthanedisulfonate de 5-[4-[2-(N-méthyl-N-(2-pyridyl)amino)éthoxy]benzyl]thiazolidine-2,4-dione (2:1) suivant l'une quelconque des revendications 10 à 13, **caractérisé en ce qu'**il présente :
(i) un spectre infrarouge essentiellement suivant la figure 5 ; et
(ii) un spectre Raman essentiellement suivant la figure 6 ; et
(iii) un diagramme de diffraction des rayons X sur poudre (XRPD) essentiellement suivant le tableau 2 ou la figure 7 ; et
(iv) un spectre de ¹³C-RMN à l'état solide essentiellement suivant la figure 8.

15. Sel suivant la revendication 1, consistant en le 1,2-éthanedisulfonate de 5-[4-[2-(N-méthyl-N-(2-pyridyl)-amino)éthoxy]benzyl]thiazolidine-2,4-dione sous forme du sel 1:1.

16. Composé suivant l'une quelconque des revendications 1 à 15, ou un de ses produits de solvatation, sous forme isolée.

17. Composé suivant l'une quelconque des revendications 1 à 15, ou un de ses produits de solvatation, sous une forme substantiellement pure.

18. Composé suivant l'une quelconque des revendications 1 à 15, ou un de ses produits de solvatation, sous forme cristalline.

19. Procédé pour la préparation d'un composé suivant l'une quelconque des revendications 1 à 15, ou d'un de ses produits de solvatation, **caractérisé en ce que** de la 5-[4-[2-(N-méthyl-N-(2-pyridyl)amino)éthoxy]benzyl]thiazolidine-2,4-dione (composé (I)) ou un de ses sels, de préférence à l'état dispersé ou dissous dans un solvant convenable, est amenée à réagir avec une source convenable d'ion éthanedisulfonate ; et le sel éthanedisulfonate ou un de ses produits de solvatation est recueilli.

20. Sel de 5-[4-[2-(N-méthyl-N-(2-pyridyl)amino)-éthoxy] benzyl] thiazolidine-2, 4-dione et d'acide 1, 2-éthanedisulfonique ou un de ses produits de solvatation pharmaceutiquement acceptables, suivant la revendication 1, destiné à être utilisé comme substance thérapeutique active.

21. Sel de 5-[4-[2-(N-méthyl-N-(2-pyridyl)amino)-éthoxy]benzyl]thiazolidine-2,4-dione et d'acide 1,2-éthanedisulfonique ou un de ses produits de solvatation pharmaceutiquement acceptables, suivant la revendication 1, destiné à être utilisé dans le traitement et/ou la prophylaxie du diabète sucré, d'affections associées au diabète sucré et de certaines de ses complications.

22. Utilisation d'un sel de 5-[4-[2-(N-méthyl-N-(2-pyridyl)amino)éthoxy]benzyl]thiazolidine-2,4-dione et d'acide 1,2-éthanedisulfonique ou un de ses produits de solvatation pharmaceutiquement acceptables, suivant la revendication 1, pour la production d'un médicament destiné au traitement et/ou à la prophylaxie du diabète sucré, d'affections associées au diabète sucré et de certaines de ses complications.

23. Utilisation suivant la revendication 22, dans laquelle le médicament comprend, en association, un ou plusieurs autres agents antidiabétiques.
